# EUROPÄISCHE PATENTSCHRIFT

(11) **EP 3 142 671 B1**
(45) Veröffentlichungstag und Bekanntmachung des Hinweises auf die Patenterteilung: **22.12.2021**
(21) Anmeldenummer: 15724579.6
(22) Anmeldetag: 15.05.2015
(51) Int. Cl.: A61K 31/7004, A61K 31/7048, A61P 3/04, A61P 3/10

(54) **KOMBINATION VON BIOLOGISCH AKTIVEN SUBSTANZEN ZUR BEHANDLUNG VON HYPERGLYKÄMISCHEN ERKRANKUNGEN**
COMBINATION OF BIOLOGICALLY ACTIVE SUBSTANCES FOR TREATING HYPERGLYCEMIC DISEASES
COMBINAISON DE SUBSTANCES BIOLOGIQUES ACTIVES DESTINÉE AU TRAITEMENT DE PATHOLOGIES HYPOGLYCÉMIQUES

(30) Priorität: 16.05.2014 EP 14168754
(43) Veröffentlichungstag der Anmeldung: 22.03.2017
(73) Patentinhaber: BioActive Food GmbH, 23795 Bad Segeberg (DE)
(72) Erfinder: VOLLERT, Henning, 23795 Bad Segeberg (DE)
(74) Vertreter: Uexküll & Stolberg
(86) Internationale Anmeldenummer: PCT/EP2015/060743
(87) Internationale Veröffentlichungsnummer: WO 2015/173383

(56) Entgegenhaltungen:
- EP-A1- 2 679 229
- WO-A1-2012/168108
- WO-A2-2007/124102
- KI WON LEE ET AL: "Major Phenolics in Apple and Their Contribution to the Total Antioxidant Capacity", JOURNAL OF AGRICULTURAL AND FOOD CHEMISTRY, Bd. 51, Nr. 22, 1. Oktober 2003 (2003-10-01), Seiten 6516-6520, XP055133815, ISSN: 0021-8561, DOI: 10.1021/jf034475w
- EHERENKRANZ J R L ET AL: "Phlorizin: a review", DIABETES/METABOLISM RESEARCH AND REVIEWS, WILEY, LONDON, GB, Bd. 21, Nr. 1, 1. Januar 2005 (2005-01-01) , Seiten 31-38, XP009091215, ISSN: 1520-7552, DOI: 10.1002/DMRR.532
- Narasimhanaidu Kamalakkannan ET AL: "Antihyperglycaemic and antioxidant effect of rutin, a polyphenolic flavonoid, in streptozotocin-induced diabetic wistar rats", Basic & Clinical Pharmacology & Toxicology, 1. Januar 2006 (2006-01-01), Seiten 97-103, XP055133712, Denmark DOI: 10.1111/j.1742-7843.2006.pto_241.x Gefunden im Internet: URL:http://www.ncbi.nlm.nih.gov/pubmed/164 33898
- JI-HYE KIM ET AL: "Quercetin attenuates fasting and postprandial hyperglycemia in animal models of diabetes mellitus", NUTRITION RESEARCH AND PRACTICE, Bd. 5, Nr. 2, 1. Januar 2011 (2011-01-01), Seite 107, XP055133715, ISSN: 1976-1457, DOI: 10.4162/nrp.2011.5.2.107
- YANLING ZHANG ET AL: "Flavonol kaempferol improves chronic hyperglycemia-impaired pancreatic beta-cell viability and insulin secretory function", EUROPEAN JOURNAL OF PHARMACOLOGY, ELSEVIER SCIENCE, NL, Bd. 670, Nr. 1, 17. August 2011 (2011-08-17), Seiten 325-332, XP028312085, ISSN: 0014-2999, DOI: 10.1016/J.EJPHAR.2011.08.011 [gefunden am 2011-09-02]
- ZANG YANQING ET AL: "Anti-diabetic effects of a kaempferol glycoside-rich fraction from unripe soybean (Edamame, Glycine max L. Merrill. 'Jindai') leaves on KK-A(y) mice.", BIOSCIENCE, BIOTECHNOLOGY, AND BIOCHEMISTRY 2011, Bd. 75, Nr. 9, 7. November 2011 (2011-11-07), Seiten 1677-1684, XP055133718, ISSN: 1347-6947

## Beschreibung

Die vorliegende Erfindung betrifft eine synergistisch wirkende Zusammensetzung, die (a) Phlorizin und (b) mindestens einen näher definierten Inhibitor des Enzyms Laktase-Phlorizin-Hydrolase umfasst, zur Verwendung in einem Verfahren zur Behandlung und/oder Prophylaxe einer hyperglykämischen Erkrankung, wie z.B. Fettsucht, Diabetes oder einer von Diabetes verursachten Folgeerkrankung. Die Erfindung betrifft ferner die Verwendung einer solchen Zusammensetzung zur Herstellung einer pharmazeutischen Zusammensetzung und/oder eines Nahrungsergänzungsmittels. Die Erfindung betrifft darüber hinaus pharmazeutische Zusammensetzungen, diätetische Lebensmittel und Nahrungsergänzungsmittel, die eine Zusammensetzung gemäß der vorliegenden Erfindung umfassen.

### GEBIET DER ERFINDUNG

Die Zahl der Diabetes-Toten ist nach Angaben des Statistischen Bundesamts seit 1980 um 29 Prozent gestiegen. Diabetes mellitus ist damit verantwortlich für knapp drei Prozent aller Sterbefälle (1980: zwei Prozent) in Deutschland. Parallel dazu steigt die Zahl der übergewichtigen Menschen in Deutschland stark an. Jeder zweite Deutsche ist bereits übergewichtig, jeder fünfte sogar fettleibig. Bei diesen Menschen ist das Risiko, an Diabetes zu erkranken, dramatisch erhöht.

Aktuelle Hochrechnungen gehen davon aus, dass bis zu 7% der Deutschen (Hauner H. Dtsch Med Wochenschr 2005; 130 Suppl 2:S.64-65), d.h. rund 6 Millionen Menschen, wegen Diabetes in Behandlung sind. Die Anzahl der Neuerkrankungen nimmt dabei mit steigendem Alter zu. In der Altersgruppe der über 60-jährigen liegt der Anteil der an Diabetes Erkrankten bereits bei 18-28%. Parallel dazu sinkt das Manifestationsalter betroffener Typ-2-Diabetiker stetig. Typ-2-Diabetes ist mit rund 11% die vierthäufigste Diagnose der hausärztlichen Internisten und mit rund 8% die fünfthäufigste Diagnose aller Allgemeinärzte (Wittchen HU, (HYDRA)-Studie, Fortschr Med Orig 2003; 121). Aktuelle Schätzungen der WHO gehen davon aus, dass sich die Zahl der an Diabetes Erkrankten bis zum Jahr 2025 verdoppeln wird (World Health Organization, Diabetes mellitus, Fact sheet No. 138, 2002).

Mit der Nahrung wird eine Vielzahl an Fetten, Kohlehydraten und Proteinen aufgenommen, die den menschlichen Körper mit der benötigten Energie versorgen. Eine zu starke Aufnahme und insbesondere eine zu schnelle Aufnahme von Zuckern stellt eine starke Belastung des Stoffwechsels dar und gilt als ein wesentlicher Risikofaktor für Diabetes, Übergewicht und Fettleibigkeit. Es besteht daher ein anhaltender Bedarf an neuen Mitteln für die Behandlung von hyperglykämischen Erkrankungen wie Diabetes und Fettleibigkeit.

Zahlreiche Verbindungsklassen sind für die Behandlung des Diabetes mellitus, insbesondere des Diabetes mellitus Typ II, vorgeschlagen worden. In verschiedenen Internationalen Anmeldungen sind beispielsweise Substanzen vorgeschlagen worden, die den Natrium-abhängigen Glucose-Transporter 2 (SGLT-2) hemmen (siehe z.B. WO 98/31697, WO 02/083066, WO 03/099836 und WO 01/31697). Dieses Transportprotein resorbiert im proximalen Tubulus der Niere Glucose und Natrium aus dem Primärharn. Spezifische SGLT-2-Inhibitoren sind in der Lage, die Aufnahme von Glucose in den Nierentubuli zu verhindern, was zur Wiederherstellung von normalen Plasmaglucosespiegeln führt. Eine langfristige Behandlung mit SGLT-2-Inhibitoren von mehr als 6 Monaten hat im Tierversuch zu einer verstärkten Insulin-Empfindlichkeit, einer verbesserten Insulin-Reaktion und zu einer verzögerten Ausbildung von Diabetes-bedingten Komplikationen geführt. Die Inhibierung des Transporters SGLT-2 beruht auf einer verstärkten konzentrationsabhängigen Ausscheidung von Glucose, was zu einer Senkung des Blutzuckers im Körper führt. Dabei wird jedoch keine Hypoglykämie verursacht. Der Mechanismus der Blutzuckersenkung ist unabhängig von einer ggf. bestehenden Insulinresistenz oder einer mangelnden Insulinproduktion durch die Betazellen der Bauchspeicheldrüse. Neben SGLT-2 gibt es einen weiteren Natrium-abhängigen Glucose-Transporter, SGLT-1, der ebenfalls mit der Behandlung des Diabetes in Verbindung gebracht wird. SGLT-1 wird im Dünndarm sowie im S3-Segment des proximalen Nierentubulus gefunden. Er ist für die aktive Aufnahme von Glucose verantwortlich. Im Vergleich zu SGLT-2 weist SGLT-1 für einige Zucker eine veränderte Substratspezifität auf. Verschiedene Präparate, die zu einer Hemmung von SGLT-1 und/oder SGLT-2 führen, befinden sich derzeit in klinischen Erprobungen.

Ki Won Lee et al. (2003), Journal of Agricultural and Food Chemistry, Bd. 51, 22, Seiten 6516-6520, beschreibt den Gehalt von Phlorizin und Quercetinglycosiden in Äpfeln.

WO 2007/124102 A2 offenbart Mischungen aus Phlorizin und Apfelextrakt. Die Mischungen werden u.a. zur Gewichtsreduktion sowie zur Behandlung von Diabetes mellitus Typ 2 vorgeschlagen.

Die Europäische Patentanmeldung 12174534.3 beschreibt die Verwendung einer Zusammensetzung, die einen Extrakt einer Pflanze der Gattung *Brassica* in Kombination mit Phlorizin oder einen Phlorizin-haltigen Extrakt enthalten. Die Zusammensetzung wird zur Behandlung von hyperglykämischen Erkrankungen vorgeschlagen.

### BESCHREIBUNG DER ERFINDUNG

Im Rahmen der vorliegenden Erfindung wurde nunmehr festgestellt, dass eine vorteilhafte, synergistische Wirkung auf den Blutzuckerspiegel erhalten werden kann, wenn neben einer Hemmung des Natrium-abhängigen Glucosetransporter SGLT1 durch Phlorizin auch eine Hemmung des Enzyms Laktase-Phlorizin-Hydrolase (LPH) durch einen wie vorliegend beschriebenen Inhibitor erfolgt. Eine kombinierte Gabe von Phlorizin und einem wie vorliegend definierten Inhibitor der LPH führt zu einer deutlich geringeren Erhöhung der Blutglucosekonzentration nach der Aufnahme von Lebensmitteln mit einem hohen Anteil Kohlenhydrate, wie z.B. Stärke, und eignet sich daher in hohem Maße für die Behandlung und/oder Prophylaxe einer hyperglykämischen Stoffwechselerkrankung wie Diabetes und/oder Übergewicht.

Das Enzym LPH ist im Stand der Technik hinreichend beschrieben und wird im Darm von Säugetieren synthetisiert. Das Enzym ist als integrales Membranprotein in der Bürstensaummembran der Hauptzellen des Zottenepithels des Dünndarms aller Säugetiere vorhanden. Es katalysiert im intestinalen Lumen die Spaltung von beta-glycosidischen Bindungen in Kohlenhydraten, wie z.B. Laktose. Neben der Hydrolysierung von Phlorizin ist die LPH auch in der Lage, das Disaccharid Lactose in die Monosaccharide Galactose und Glucose aufzuspalten. Diese beiden verschiedenen Aktivitäten des Enzyms finden an unterschiedlichen reaktiven Zentren der LPH statt. Erfindungsgemäß ist ein Inhibitor der LPH insbesondere in der Lage, die Phlorizin-spaltende enzymatische Aktivität der LPH, vorzugsweise der humanen LPH, zu hemmen. Dabei ist es bevorzugt, dass der Inhibitor diese Aktivität der LPH um mindestens 5%, 10%, 15%, stärker bevorzugt mindestens 20%, 25%, 30% oder stärker bevorzugt mindestens 40%, 50% 60%, 70% oder 80% reduziert. Dem Fachmann sind Verfahren bekannt, mittels derer die Aktivität von LPH und entsprechend auch ihre Inhibition bestimmt werden kann. Solche Verfahren werden darüber hinaus in den Beispielen der vorliegenden Anmeldung beschrieben.

Bei dem oder den im Rahmen der vorliegenden Erfindung verwendeten Inhibitoren der LPH handelt es sich um Flavonolglycoside, die ein Quercetin- oder ein Kaempferol-Grundgerüst aufweisen und über eine glycosidische Bindung mit mindestens einer Zucker-Gruppe verbunden sind. Folglich handelt es sich bei den erfindungsgemäß vorgeschlagenen Inhibitoren um Quercetin- oder Kaempferol-Glycoside.

In einem ersten Aspekt betrifft die Erfindung Zusammensetzungen zur Verwendung in einem Verfahren zur Behandlung und/oder Prophylaxe einer hyperglykämischen Erkrankung, wobei die Zusammensetzungen mindestens die folgenden Bestandteile enthalten: (a) Phlorizin und (b) mindestens einen Inhibitor der Laktase-Phlorizin-Hydrolase, wobei der Inhibitor ein Glycosid eines Flavonols mit der Struktur gemäß Formel I 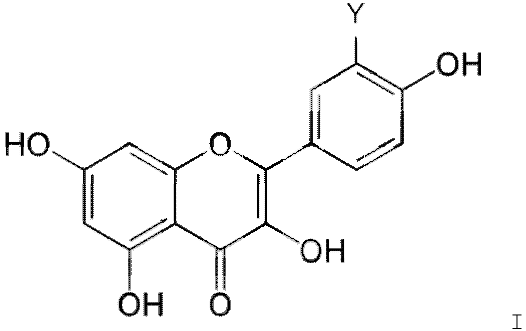 ist, in der Y ausgewählt ist aus der Gruppe bestehend aus H und OH; und wobei der Inhibitor mindestens eine glycosidisch an das Flavonol gebundene Zucker-Gruppe aufweist, und wobei die Zusammensetzung von 0,1 bis 10 g des Inhibitors der Laktase-Phlorizin-Hydrolase und von 0,01 bis 10 g Phlorizin umfasst.

Flavonole sind eine Untergruppe der chemischen Gruppe der Flavonoide. Flavonoide zählen zur Gruppe der sekundären Pflanzenstoffe, und sie stellen eine umfangreiche Stoffklasse polyphenolischer Verbindungen dar, die in Lebensmitteln pflanzlicher Herkunft verbreitet vorkommen.

Im Rahmen der Erfindung wurde nunmehr festgestellt, dass Glycoside eines Flavonols der Formel I wirksame Inhibitoren der LPH sind. Die oben angegebene Formel I zeigt die Grundstruktur der Flavonole Kaempferol und Quercetin. Sofern Y ein H ist, so entspricht Formel I Kaempferol. Ist Y eine OH-Gruppe, so entspricht Formel I Quercetin. Es ist somit erfindungsgemäß bevorzugt, dass der Inhibitor der LPH ein Glycosid von Kaempferol oder ein Glycosid von Quercetin ist.

Die als Inhibitor der LPH wirksame Substanz der Formel I weist mindestens eine glycosidisch an das Flavonol gebundene Zucker-Gruppe auf. Es handelt sich daher bei dem LPH-Inhibitor um ein Glycosid der Formel I. Glycoside im Sinne der vorliegenden Erfindung sind Verbindungen, die einen glycosidisch gebundenen Zuckerrest umfassen und die allgemeine Struktur R-O-Z besitzen, wobei Z der Zuckerrest und R das Aglycon ist. Jede Art von Zucker, der über eine glycosidische Bindung an Formel I binden kann, kann in den erfindungsgemäßen LPH Inhibitoren eingesetzt werden. Die glycosidisch gebundene Gruppe kann z.B. ein Mono- oder ein Polysaccharid, wie z.B. ein Disaccharid oder ein Trisaccharid sein.

Es ist erfindungsgemäß bevorzugt, dass die glycosidisch gebundene Gruppe ein Monosaccharid ist. So kann es sich z.B. bei dem Monosaccharid, welches glycosidisch an das Flavonol gemäß Formel I gebunden ist, um ein Monosaccharid handeln, das ausgewählt ist aus der Gruppe bestehend aus Glucose, Fructose, Rhamnose, Glucopyranose, Mannose, und Galactose. Das Monosaccharid ist vorzugsweise Glucose. Somit bezieht sich die vorliegende Erfindung in einer bevorzugten Ausführungsform auf eine Zusammensetzung zur Verwendung in einem Verfahren zur Behandlung und/oder Prophylaxe einer hyperglykämischen Erkrankung, wobei die Zusammensetzung (a) Phlorizin und (b) mindestens einen Inhibitor der LPH umfasst, wobei der Inhibitor ein Glycosid eines Flavonols der Formel I ist, und wobei Y ausgewählt ist aus der Gruppe bestehend aus H und OH; und wobei der Inhibitor mindestens eine glycosidisch an das Flavonol gebundene Glucose-Gruppe aufweist. Demgemäß handelt es sich in dieser Ausführungsform bei dem Inhibitor der LPH um ein Glucosid. Ein Glucosid ist eine Verbindung, die einen glycosidisch gebundenen Glucoserest umfasst und die allgemeine Struktur R-O-Glc besitzt, wobei Glc ein Glucoserest und R das Aglycon ist.

In einer weiteren bevorzugten Ausführungsform handelt es sich bei der Zucker-Gruppe, die glycosidisch an die Verbindung der Formel I gebunden ist, um ein Polysaccharid. Geeignet Polysaccharide umfassen z.B. Di-, Tri-, Tetra- und Pentasaccharide. Geeignete Disaccharide sind z.B. Cellobiose, Gentiobiose, Isomaltose, Isomaltulose, Lactose, Lactulose, Laminaribiose, Maltose, Maltulose, Melibiose, Neohesperidose, Neotrehalose, Nigerose, Rutinose, Sambubiose, Sophorose, Saccharose, und Trehalose. Geeignete Trisaccharide sind z.B. Fucosidolactose, Gentianose, Isokestose (1-Kestose), Kestose (6-Kestose), Maltotriose, Manninotriose, Melezitose, Neokestose, Panose, Raffinose, und Umbelliferose. In einer bevorzugten Ausführungsform ist das an das Flavonol gebundene Polysaccharid Rutinose.

Die glycosidisch gebundene Zucker-Gruppe ist vorzugsweise beta-glycosidisch an das Flavonol gebunden. Dabei ist es besonders bevorzugt, dass die glycosidische Bindung der Zucker-Gruppe über eine der in Formel I gezeigten OH-Gruppen in Position 3', 4', 3, 5 oder 7 erfolgt. Die Nummerierung der Positionen in dem Flavonol folgt der allgemein bekannten chemischen Nomenklatur, wie es in der folgenden Formel II angegeben ist:

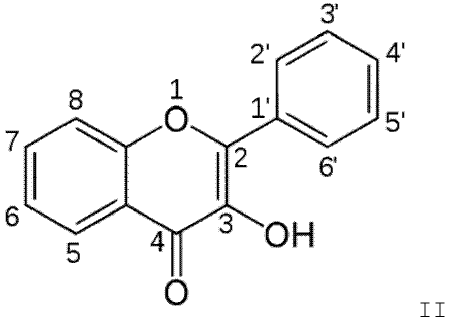

In einer bevorzugten Ausführungsform befindet sich die glycosidisch gebundene Gruppe des Inhibitors in Position 7 des Flavonols. In einer weiteren bevorzugten Ausführungsform befindet sich die glycosidisch gebundene Gruppe des Inhibitors in Position 3 des Flavonols. In einer noch weiteren bevorzugten Ausführungsform umfasst der Inhibitor nur eine einzige glycosidisch gebundene Zucker-Gruppe, die sich in Position 3 des Flavonols befindet. In einer noch weiteren bevorzugten Ausführungsform umfasst der Inhibitor nur eine einzige glycosidisch gebundene Zucker-Gruppe, die sich in Position 7 des Flavonols befindet. Das bedeutet, dass der Inhibitor in diesen Ausführungsformen zusätzlich zu der glycosidisch gebundenen Gruppe in Position 3 oder 7 des Flavonols keine weitere glycosidisch gebundene Gruppe enthält.

Erfindungsgemäß kann der LPH-Inhibitor auch mehrere Zucker-Gruppen umfassen, die jeweils über eine glycosidische Bindung an das Flavonol gemäß Formel I gebunden sind. Der LPH-Inhibitor kann z.B. 2, 3, 4, 5 oder mehr derartig gebundene Zucker-Gruppen umfassen. Diese glycosidisch gebundenen Zucker werden ebenfalls vorzugsweise über eine der in Formel I gezeigten OH-Gruppen in Position 3', 4', 3, 5 oder 7 gebunden sein.

Bei dem Inhibitor der LPH kann es sich insbesondere um eines der folgenden Moleküle handeln: Quercetin-7-O-Glucosid, Kaempferol-7-O-Glucosid, Quercetin-3-O-rhamnosid, Quercetin-3-O-glucopyranosid, Kaempferol-3-O-Glucosid, Kaempferol-3-O-rutinosid, Quercetin-3-galactosid, Quercetin-4'-Glucosid, Quercetin-3-O-rutinosid (Quercetin-3-O-glucorhamnosid), Kaempferol-3,7-di-O-α-L-rhamnosid, Kaempferol-3-(2G-xylosyl-rutinosid), 3-Glucosylquercetin, 3-Glucosylkaempferol, Dihydrokaempferol-3-rhamnosid, Quercetin-3,4-O-Glucosid, 4'-Methylkaempferol, Kaempferol-3-O-feruloyl-diglucosid-7-O-glucosid, Kaempferol-3-O-hydroxyferuloyl-diglucosid, Kaempferol-3-O-disinapoyl-triglucosid-7-O-diglucosid, Kaempferol-3-O-sinapoyl-triglucosid, Kaempferol-3-O-sinapoyl-diglucosid, Kaempferol-3-O-disinapoyl-triglucosid-7-O-glucosid, Kaempferol-3-O-diglucosid-7-O-diglucosid, Kaempferol-3-O-triglucosid-7-O-glucosid, Kaempferol-3-O-glucosid-7-O-glucosid, Kaempferol-3-O-triglucosid, Kaempferol-3-O-diglucosid, Kaempferol-3-O-glucosid, und ähnliche.

In einer besonders bevorzugten Ausführungsform ist der LPH-Inhibitor Quercetin-7-O-Glucosid. In einer anderen Ausführungsform ist der LPH Inhibitor Kaempferol-7-O-Glucosid.

Darüber hinaus können die erfindungsgemäßen LPH Inhibitoren auch SGLT1 Inhibitoren sein, so dass sie eine duale Wirkung entfalten. Beispiele für solche dualen Inhibitoren sind z.B. Quercetin-7-O-Glucosid und Kaempferol-7-O-Glucosid.

Die glycosidisch an das Flavonol gebundene Zucker-Gruppe kann weitere Modifikationen aufweisen. So ist die Zucker-Gruppe in einer Ausführungsform acyliert, d.h. die Zucker-Gruppe umfasst eine oder mehrere Acylgruppen. Bevorzugte, acylierte Inhibitoren der Laktase-Phlorizin-Hydrolase sind z.B. ausgewählt aus der Gruppe bestehend aus Kaempferol-3-O-hydroxyferuloyl-tetraglucosid, Kaempferol-3-O-hydroxyferuloyl-diglucosid-7-O-hydroxyferuloyl-diglucosid, Kampferol-3-O-hydroxyferuloyl-di-glucosid-7-O-glucosid, Kaempferol-3-O-sinapoyl-diglucosid-7-O-diglucosid und Kaempferol-3-O-sinapoyl-diglucosid-7-O-glucosid. In einer besonders bevorzugten Ausführungsform ist der Inhibitor Kaempferol-3-O-sinapoyl-diglucosid-7-O-diglucosid.

Es ist erfindungsgemäß bevorzugt, dass es sich bei der glycosidisch an das Flavonol gebundene Zucker-Gruppe um ein acyliertes Polysaccharid handelt.

Darüber hinaus kann eine an das Flavonol gebundene Zucker-Gruppe ein Zimtsäurederivat sein, d.h. die Zucker-Gruppe umfasst eine oder mehrere von der Zimtsäure abgeleitete Gruppen, wie z.B. eine Hydroxzimtsäuregruppe, wie eine Sinapinsäuregruppe (3,5-Dimethoxy-4-hydroxy-zimtsäure), α-Cyano-4-hydroxy-zimtsäuregruppe (HCCA), Ferulasäuregruppe (4-Hydroxy-3-methoxyzimtsäure) und Kaffeesäuregruppe. In dieser Ausführungsform liegt die Zucker-Gruppe z.B. als Sinapoyl-Glycosid oder als Feruloyl-Glycosid vor.

Die erfindungsgemäß verwendeten Zusammensetzungen umfassen neben einem oder mehreren Inhibitoren der LPH das Flavonoid Phlorizin. Phlorizin hemmt den Transporter SGLT 1 *in vitro* (Kottra et al. (2007), J Pharmacol Exp Ther, 322 (2) :829-35). Es ist ferner seit langer Zeit bekannt, dass Phlorizin auf die Glucoseausscheidung der Niere wirkt, wenn es intravenös appliziert wird. Allerdings ist die Wirkung schwach, wenn Phlorizin oral eingenommen wird. Dies ist vermutlich teilweise auf einen enzymatischen Abbau des Phlorizins im Darm zurückzuführen, der z.B. durch das Enzym LPH katalysiert wird (Birkenmeier & Alpers (1974), Biochim Biophys Acta., 350(1):100-12). Nach Einnahme der erfindungsgemäß verwendeten Zusammensetzung wird der enzymatische Abbau des Phlorizins im Darm durch den oder die oben definierten Inhibitoren der LPH gehemmt, wodurch das Phlorizin seine inhibitorischen Eigenschaften auf die Glucoseaufnahme (SGLT 1) entfaltet.

Die erfindungsgemäß verwendeten Zusammensetzungen enthalten eine Menge an Phlorizin, die wirksam ist, um nach oraler Gabe den Glucose-Transporters SGLT-1 zu hemmen und/oder die Zuckeraufnahme aus dem Darm zu verlangsamen. In einer bevorzugten Ausführungsform wird die Aktivität des im Darm befindlichen SGLT-1-Transporters durch die Zusammensetzungen um mindestens etwa 1%, 2%, 3%, 4%, 5%, 10%, 20%, 30%, 40%, 50%, 60%, 70%, 80%, 90% oder mehr gegenüber dem unbehandelten Zustand verringert. In einer weiteren bevorzugten Ausführungsform führt die erfindungsgemäß verwendete Zusammensetzung nach Gabe zu einer Zuckeraufnahme aus dem Darm die gegenüber dem unbehandelten Zustand um mindestens etwa 1%, 2%, 3%, 4%, 5%, 10%, 20%, 30%, 40%, 50%, 60%, 70%, 80%, 90% oder mehr verringert ist.

Der Fachmann ist problemlos in der Lage, die für die jeweils zu erreichende (therapeutische) Wirkung erforderliche Menge an Phlorizin und LPH-Inhibitor durch einfache Versuchsreihen zu ermitteln. Hierbei wird der Fachmann verschiedene Faktoren berücksichtigen, wie z.B. die Art und Menge des oder der in der Zusammensetzung vorhandenen Inhibitoren der LPH, die Art und Menge der zusätzlich in den Zusammensetzungen vorhandenen Komponenten, Alter und Gewicht der Person, an die die Verabreichung der Zusammensetzung erfolgt, usw.

Die Menge an Phlorizin in den erfindungsgemäß verwendeten Zusammensetzungen ist vorzugsweise größer als 1 ppm (parts per million) oder 0,0001 % (w/w). Es ist bevorzugt, dass die Menge an Phlorizin in der Zusammensetzung größer ist als 10 ppm, größer als 100 ppm, größer als 1000 ppm, größer als 10⁴ ppm, größer als 5*10⁴ ppm oder größer als 10⁵ ppm. Anders ausgedrückt enthält die erfindungsgemäß verwendete Zusammensetzung mehr als 0,0001% (w/w) mehr als 0,001%, vorzugsweise mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, mehr als 14%, oder mehr als 15% Phlorizin. Ebenfalls bevorzugt ist es, dass die erfindungsgemäß verwendete Zusammensetzung mehr als 20% (w/w), mehr als 25%, mehr als 30%, mehr als 35%, mehr als 40%, mehr als 45%, mehr als 50%, mehr als 55%, oder mehr als 60% Phlorizin umfasst. In einer besonders bevorzugten Ausführungsform enthalten die erfindungsgemäß verwendeten Zusammensetzungen zwischen 1-95% (w/w), wie z.B. 5-80%, 10-70%, 15-60%, 20-50%, oder 30-40% Phlorizin, oder wie z.B. 5-95%, 10-95%, 15-95%, 20-95%, oder 30-95% Phlorizin.

Die erfindungsgemäß verwendete Zusammensetzung wird so formuliert, dass die jeweilige Verabreichungseinheit (z.B. eine Kapsel, eine Tablette oder eine definierte Menge einer Flüssigkeit) eine Menge von 0,01 bis 10 g Phlorizin enthält, z.B. mehr als 0,05 g, mehr als 0,1 g, mehr als 0,2 g, mehr als 0,3 g, mehr als 0,4 g, mehr als 0,5 g, mehr als 0,75 g, mehr als 1 g, mehr als 2 g, mehr als 3 g, mehr als 4 g oder mehr als 5 g.

Die erfindungsgemäß verwendeten Zusammensetzungen enthalten ferner eine Menge an LPH-Inhibitor, die wirksam ist, um nach oraler Gabe die Laktase-Phlorizin-Hydrolase zu hemmen. In einer bevorzugten Ausführungsform wird die Aktivität der im Darm befindlichen LPH durch die Zusammensetzungen um mindestens etwa 1%, 2%, 3%, 4%, 5%, 10%, 20%, 30%, 40%, 50%, 60%, 70%, 80%, 90% oder mehr gegenüber dem unbehandelten Zustand verringert.

Die Menge an LPH-Inhibitor in den erfindungsgemäß verwendeten Zusammensetzungen ist vorzugsweise ebenfalls größer als 1 ppm (parts per million) oder 0,0001 % (w/w). Es ist bevorzugt, dass die Menge an LPH Inhibitor in der Zusammensetzung größer ist als 10 ppm, größer als 100 ppm, größer als 1000 ppm, größer als 10⁴ ppm, größer als 5*10⁴ ppm oder größer als 10⁵ ppm. Anders ausgedrückt enthält die erfindungsgemäß verwendete Zusammensetzung mehr als 0,0001% (w/w), mehr als 0,001%, vorzugsweise mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, mehr als 14%, oder mehr als 15% LPH Inhibitor. Ebenfalls bevorzugt ist es, dass die erfindungsgemäß verwendete Zusammensetzung mehr als 20% (w/w), mehr als 25%, mehr als 30%, mehr als 35%, mehr als 40%, mehr als 45%, mehr als 50%, mehr als 55%, oder mehr als 60% des oder der hierin definierten LPH Inhibitors umfasst. In einer besonders bevorzugten Ausführungsform enthalten die erfindungsgemäß verwendeten Zusammensetzungen zwischen 1-95% (w/w), wie z.B. 5-80%, 10-70%, 15-60%, 20-50% oder 30-40% LPH-Inhibitor, oder wie z.B. 5-95%, 10-95%, 15-95%, 20-95%, oder 30-95% LPH-Inhibitor.

In einer Ausführungsform handelt es sich bei dem LPH Inhibitor, der in den obigen Mengen in der erfindungsgemäß verwendeten Zusammensetzung vorliegt, um Kaempferol-7-O-Glucosid. In einer anderen Ausführungsform handelt es sich bei dem LPH Inhibitor, der in den obigen Mengen in der erfindungsgemäß verwendeten Zusammensetzung vorliegt, um Quercetin-7-O-Glucosid.

Die erfindungsgemäß verwendete Zusammensetzung wird so formuliert, dass die jeweilige Verabreichungseinheit (z.B. eine Kapsel, ein Pulver, eine Tablette oder eine definierte Menge einer Flüssigkeit) eine Menge von 0,1 bis 10 g LPH-Inhibitor enthält, z.B. mehr als 0,2 g, mehr als 0,3 g, mehr als 0,4 g, mehr als 0,5 g, mehr als 0,75 g, mehr als 1 g, mehr als 2 g, mehr als 3 g, mehr als 4 g oder mehr als 5 g.

Besonders bevorzugt ist es, dass die erfindungsgemäß verwendete Zusammensetzung für die orale Verabreichung formuliert ist und von 0,1 bis 10 g LPH-Inhibitor, vorzugsweise von 1-3 g LPH-Inhibitor enthält sowie von 0,01 bis 10 g Phlorizin, vorzugsweise von 1-3 g Phlorizin.

In einer weiteren Ausführungsform der Erfindung beträgt die Menge an Phlorizin in der Zusammensetzung mindestens 0,1 g, und die Menge an LPH-Inhibitor gemäß der obigen Formel I in der Zusammensetzung beträgt ebenfalls mindestens 0,1 g. In einer noch weiteren Ausführungsform der Erfindung beträgt die Menge an Phlorizin in der Zusammensetzung mindestens 1 g, und die Menge an Flavonolglycosid gemäß der obigen Formel I in der Zusammensetzung beträgt ebenfalls mindestens 1 g. In einer noch weiteren Ausführungsform beträgt die Menge an Phlorizin in der Zusammensetzung zwischen 0,1 und 1 g, und die Menge an LPH-Inhibitor gemäß der obigen Formel I in der Zusammensetzung beträgt ebenfalls zwischen 0,1 und 1 g.

Phlorizin und LPH-Inhibitor(en) können in jeglichem Mischungsverhältnis verwendet werden. Die Mengen von Phlorizin und LPH-Inhibitor in den erfindungsgemäß verwendeten Zusammensetzungen werden jedoch vorzugsweise vom Fachmann so kombiniert, dass eine optimale Wirkung erzielt wird, d.h. sowohl LPH als auch SGLT1 effizient inhibiert werden. In einer Ausführungsform enthält die erfindungsgemäß verwendete Zusammensetzung Phlorizin und LPH-Inhibitor(en) im Verhältnis von ungefähr 20:1, 10:1, 5:1, 4:1, 3:1, 2:1, 1:1, 1:2, 1:3, 1:4, 1:5, 1:10 oder 1:20. Ein Mischungsverhältnis zwischen Phlorizin und dem oder den LPH-Inhibitoren von 1:1 ist besonders bevorzugt.

In einer bevorzugten Ausführungsform der Erfindung handelt es sich bei der erfindungsgemäß verwendeten Zusammensetzung nicht um eine Pflanze oder einen Teil einer Pflanze, wie z.B. eine Frucht. In einer besonders bevorzugten Ausführungsform wird der LPH-Inhibitor der erfindungsgemäß verwendeten Zusammensetzung nicht in Form eines pflanzlichen Extrakts zugesetzt. Stammt der LPH-Inhibitor ursprünglich aus einem Pflanzenextrakt, so wurde der Inhibitor vor seiner Verwendung in den Zusammensetzungen der vorliegenden Erfindung aus dem Extrakt aufgereinigt. Alternativ dazu kann es sich bei dem LPH-Inhibitor auch um eine Verbindung handeln, die durch chemische Synthese gewonnen wurde. Verfahren zur chemischen Synthese der oben aufgeführten inhibitorischen Flavonol-Verbindungen sind dem Fachmann bekannt und wurden in der Literatur beschrieben, siehe z.B. Hirpara et al. (2009, Anticancer Agents Med Chem, 9 (2) :138-61) .

Weiterhin ist es bevorzugt, dass die wie oben definierten LPH-Inhibitoren und Phlorizin insgesamt mehr als 70% (w/w), vorzugsweise mehr als 80%, 85%, 90%, 95% oder 99% der in der erfindungsgemäß verwendeten Zusammensetzung vorhandenen Flavonoid-Glycoside ausmachen. Dies bedeutet, dass in der erfindungsgemäß verwendeten Zusammensetzung weniger als 30% (w/w), vorzugsweise weniger als 20%, 15%, 10%, 5% oder 1% Flavonoid-Glycoside vorhanden sind, bei denen es sich nicht um die oben definierten LPH-Inhibitoren oder Phlorizin handelt. In einer besonders bevorzugten Ausführungsform enthält die erfindungsgemäß verwendete Zusammensetzung weniger als 1% (w/w) oder keine Flavonoid-Glycoside, bei denen es sich nicht um die oben definierten LPH-Inhibitoren oder Phlorizin handelt.

Es ist erfindungsgemäß besonders bevorzugt, dass sowohl das in den erfindungsgemäß verwendeten Zusammensetzungen verwendete Phlorizin als auch der LPH-Inhibitor synthetisch hergestellt sind. In einer weiteren besonders bevorzugten Ausführungsform liegen weder das Phlorizin noch der LPH-Inhibitor in Form eines Pflanzenextraktes vor.

In einer anderen Ausführungsform wird das Phlorizin dem oder den LPH-Inhibitoren in Form eines Phlorizin-haltigen pflanzlichen Extrakts zugesetzt. Der Phlorizin-haltige pflanzliche Extrakt kann z.B. aus mindestens einer Pflanze der Familie *Rosaceae* gewonnen werden. Vorzugsweise gehört die Pflanze aus der Familie *Rosaceae* dabei einer Gattung an, die ausgewählt ist aus der Gruppe bestehend aus *Malus, Pyrus* oder *Prunus.* Der Phlorizin-haltige pflanzliche Extrakt kann ferner auch aus einer Pflanze der Familie *Verbenaceae* gewonnen werden. Vorzugsweise handelt es sich bei der Pflanze aus der Familie *Verbenaceae* um eine solche der Gattung *Lippia.* Es ist bekannt, dass insbesondere Pflanzen dieser Familien Phlorizin enthalten. Der Extrakt der Pflanze aus der Familie der *Rosaceae* oder *Verbenaceae* kann dabei z.B. aus der Rinde, den Früchten oder den Blättern der Pflanze gewonnen wurde.

Pflanzen der Gattungen *Malus, Pyrus* und *Prunus* enthalten relativ hohe Mengen an Phlorizin in der Rinde, in den Früchten und in den Blättern. Insbesondere die Früchte der Gattung *Malus* (d.h. Äpfel) weisen besonders hohe Mengen an Phlorizin auf. Es ist daher besonders bevorzugt, dass das Phlorizin in der erfindungsgemäß verwendeten Zusammensetzung in Form eines Extrakts aus einer Pflanze der Gattung *Malus,* vorzugsweise in Form eines Extrakts aus Äpfeln oder der Rinde, vorliegt. Bei der Gattung *Malus* handelt es sich um eine pflanzliche Gattung in der Familie der Rosengewächse (Rosaceae). Besonders bevorzugt ist es, dass die Extrakte aus einer Pflanze der Gattung *Malus* ausgehend von Pflanzen der Spezies *Malus domestica* hergestellt werden. Der Kulturapfel *(Malus domestica* Borkh.; *Pyrus malus,* L.) ist eine wirtschaftlich sehr bedeutende Obstart, zu der zahlreiche Sorten gehören. Grundsätzlich können alle Sorten des Kulturapfels zur Herstellung der Phlorizin-haltigen Extrakte verwendet werden. Besonders bevorzugt ist es, dass die Extrakte ausgehend von einer der Sorten Red Delicious, Golden Delicious, Braeburn, Cox Orange, Finkenwerder Prinz, Fürst Blücher, Märkischer Cox oder Red Chief hergestellt werden. Neben Pflanzen der Spezies *Malus domestica* können jedoch auch andere Spezies der Gattung *Malus* Verwendung finden.

Es ist bevorzugt, dass der mindestens eine Extrakt aus der Pflanze aus der Familie der *Rosaceae* und/oder der mindestens eine Extrakt aus der Familie der *Verbenaceae* mindestens 1%, vorzugsweise mindestens 2%, 3%, 4%, 5%, 6%, 7%, 8%, 9%, 10%, 15%, 20% oder mehr Phlorizin enthält (w/w).

In einer weiteren bevorzugten Ausführungsform umfassen die Zusammensetzungen einen oder mehrere Inhibitoren der LPH und mindestens einen Extrakt aus mindestens einer Pflanze der Familie *Rosaceae* und/oder aus mindestens einer Pflanze der Familie *Verbenaceae.* In einer Ausführungsform enthalten die Zusammensetzungen einen oder mehrere, z.B. zwei, drei, vier oder mehr, der Inhibitoren der LPH und/oder mehr als einen, z.B. zwei, drei, vier oder mehr, Extrakte aus einer Pflanze der Gattung *Malus.* Besonders bevorzugt sind Zusammensetzungen, die Quercetin-7-O-Glucosid und/oder Kaempferol-7-O-Glucosid und mindestens einen Extrakt aus einer Pflanze der Gattung *Malus* enthalten. Der hier verwendete Begriff "Extrakt aus einer Pflanze" bezeichnet einen Extrakt der aus einer oder mehreren Pflanzen der gleichen Art bzw. Spezies gewonnen wird und ist nicht als auf eine einzige Pflanze beschränkt zu verstehen.

Wie vorliegend verwendet bezeichnet der Begriff "Extrakt" ein Stoffgemisch, das durch Aufschluss von Zellen einer Pflanze und Gewinnung des Zellsafts erhalten wird. Die erfindungsgemäß vorgeschlagenen Extrakte lassen sich durch eine Vielzahl von im Stand der Technik bekannten Verfahren herstellen.

In einer Ausführungsform der Erfindung wird zunächst Material einer Pflanze bereitgestellt. Als Ausgangsmaterial für das erfindungsgemäße Verfahren eignen sich Pflanzen in sämtlichen Entwicklungsstadien, vom Samen bis zur reifen Pflanze. Ferner eignen sich sämtliche Pflanzenteile, wie z.B. die Früchte, Stängel, Blätter, Rinde oder Wurzeln der Pflanze. Das Ausgangsmaterial kann eine Mischung aus verschiedenen Pflanzenteilen sein, oder nur bestimmte Pflanzenteile umfassen. Das pflanzliche Material wird in der Regel zunächst zerkleinert. Das Zerkleinern kann durch einfaches Zerstückeln der jeweiligen Pflanzenteile mit herkömmlichen Cuttern erreicht werden. Es lassen sich grundsätzlich alle Pflanzenteile als Ausgangspunkt für die Extraktbildung verwenden. Bei der Herstellung von Extrakten von Pflanzen der Gattung *Malus* haben sich die Früchte und die Rinde als besonders geeignet erwiesen. In einer weiteren bevorzugten Ausführungsform werden die Extrakte aus einer Pflanze der Gattung *Malus* aus den Früchten, d.h. den Äpfeln, oder aus der Rinde der jeweiligen Malus-Pflanze hergestellt. Vor der Zerkleinerung kann das pflanzliche Material bei Temperaturen zwischen 45°C und 100°C blanchiert werden, um bakterielle Verunreinigungen zu beseitigen und die Qualität des Aufschlusses zu verbessern. Des Weiteren wird durch das Blanchieren die Aktivität von Enzymen wie Hydrolasen, Lipasen und Oxidasen reduziert und somit die Stabilität und Qualität des Pflanzenmaterials erhöht.

In einem nächsten Schritt wird das pflanzliche Material aufgeschlossen, d.h. die zellulären Strukturen des Materials werden zerstört, sodass der Inhalt der Zellen freigesetzt wird. Der Aufschluss kann durch herkömmliche Verfahren zum Aufschluss pflanzlicher Zellen erreicht werden, beispielsweise durch wiederholtes Einfrieren und Auftauen, oder durch geeignete Apparaturen, wie z.B. durch Homogenisatoren, Hochdruckhomogenisatoren oder Ultraschallhomogenisatoren. Auch Kolloidmühlen oder French-Pressen können für den Aufschluss verwendet werden.

Darüber hinaus kann der Zellaufschluss auch enzymatisch erzielt werden. Zu diesem Zweck wird das pflanzliche Material mit geeigneten Enzymen versetzt, die zu einer Zerstörung der strukturellen Bestandteile der Zellen führen. Es hat sich gezeigt, dass eine Inkubation des pflanzlichen Materials mit Pektinase, Kollagenase, Cellulase und/oder Hemicellulasen in dieser Phase des Verfahrens besonders geeignet ist, die Zellen wirkungsvoll aufzuschließen. Die Inkubationszeit kann zwischen 30 min und 24 Stunden liegen, vorzugsweise zwischen 1 Stunde und 6 Stunden, mehr bevorzugt zwischen 90 min und 4 Stunden, z.B. 2 Stunden. Die Temperatur kann im Bereich von 20° und 60°C liegen, vorzugsweise zwischen 25°C und 45°C, z.B. bei ca. 37°C, und sie sollte vorzugsweise im Bereich des Temperaturoptimums des jeweils verwendeten Enzyms liegen. Ein geeigneter pH-Wert des Reaktionsansatzes kann unter Verwendung geeigneter Puffer, wie z.B. Phosphat-, Carbonat-, Natriumhydrogencarbonatpuffer und/oder geeigneter Säuren, wie z.B. Citronensäure, Milchsäure oder Ascorbinsäure eingestellt werden. Ein pH Wert zwischen 4 und 6, z.B. pH 5, ist für den Aufschluss besonders geeignet.

Nach Aufschluss der Zellen, kann der erhaltende Zellsaft direkt zur Hemmung des Natrium-abhängigen Glucose-Transporters 1 (SGLT-1) verwendet werden. Es ist allerdings bevorzugt, den nach dem Aufschluss der Zellen erhaltenen Pflanzensaft weiteren Reinigungsschritten zu unterziehen.

Beispielsweise kann das aus dem Zellaufschluss erhaltene Material einer Trocknung oder Gefriertrocknung mit anschließender Extraktion unterzogen werden. Hier kann beispielsweise zunächst eine Sprühtrocknung eingesetzt werden. Das getrocknete oder gefriergetrocknete pflanzliche Material kann anschließend mit einem wässrigen oder organischen Lösungsmittel extrahiert werden. Die Extraktion kann mit einem herkömmlichen Extraktionsmittel durchgeführt werden, z.B. mit Ethanol, Ethylacetat oder mit anderen organischen Lösungsmitteln. Auch eine Extraktion mit Gasen, wie z.B. mit Stickstoff ist möglich. Bei Verwendung eines flüssigen Lösungsmittels beträgt die Inkubationszeit 0,5 bis 24 Stunden, vorzugsweise 2 bis 8 Stunden. Bei Verwendung von Stickstoff erfolgt die Extraktion bei Temperaturen zwischen 4°C und 37°C in einem Zeitraum von 0,25 bis 3 Stunden, vorzugsweise in einem Zeitraum von 20 und 60 Minuten.

Die zur erfindungsgemäßen Verwendung vorgeschlagenen Zusammensetzungen lassen sich durch eine Vielzahl von im Stand der Technik bekannten Verfahren herstellen. Die LPH-Inhibitoren und Phlorizin können durch im Stand der Technik bekannte Verfahren hergestellt oder von verschiedenen Herstellern direkt erworben und in den gewünschten Mengen miteinander vermischt werden (z.B. von der Carl Roth GmbH & Co. KG, Karlsruhe, Deutschland).

In einer bevorzugten Ausführungsform sind ist das Phlorizin in dem Phlorizin-haltigen Extrakt aufkonzentriert. Verfahren zur Aufkonzentrierung dieser Substanzen sind dem Fachmann hinreichend bekannt und werden z.B. in Will et al. (2006, LWT - Food Science and Technology, 40(8):1344-1351) beschrieben. Eine Aufkonzentrierung von Phlorizin kann beispielsweise durch Verwendung von Säulenchromatographieverfahren erreicht werden. Zu diesem Zweck können z.B. verschiedene Adsorbersäulen eingesetzt werden. Die Chromatographiesäulen werden mit dem Ausgangsextrakt beladen, durch Spülen mit geeigneten Lösungen von unerwünschten Bestandteilen befreit und anschließend in aufkonzentrierter Form von der Säule eluiert. Zum Beispiel werden nach Beladen von Adsorbersäulen mit unkonzentriertem Extrakt durch Spülen mit demineralisiertem Wasser hydrophile Substanzen (Salze, Aminosäuren, Peptide, Zucker etc.) abgereichert. In dem mit 95% Ethanol eluiertem Extrakt ist anschließend Phlorizin angereichert. Die erfindungsgemäß erhältlichen Phlorizin-Extrakte können sowohl in trockener als auch in flüssiger Form hergestellt werden.

Die vorliegend beschriebenen Zusammensetzungen werden erfindungsgemäß für die Behandlung und/oder die Prophylaxe einer hyperglykämischen Erkrankung vorgeschlagen. Die Zusammensetzungen zeichnen sich durch eine synergistische Wirkung ihrer Bestandteile aus, d.h. das Zusammenwirken der einzelnen Bestandteile geht über einen rein additiven Effekt hinaus.

Erkrankungen, die sich mit Hilfe der erfindungsgemäß vorgeschlagenen Zusammensetzungen behandeln lassen, umfassen alle Krankheiten, die durch eine Hyperglykämie, d.h. durch einen erhöhten Blutzuckerspiegel gekennzeichnet sind, der den physiologisch normalen Wert von 140 mg/dl (7,8 mmol/l) übersteigt. Solche Erkrankungen umfassen insbesondere Fettsucht, Diabetes oder von Diabetes verursachte Folgeerkrankungen (wie z.B. Retinopathie, Neurophathie, Nephropathie und gestörte Wundheilung). Bei dem Diabetes, der mittels der vorliegend beschriebenen Zusammensetzungen behandelt werden soll, kann es sich um Diabetes Typ I oder Typ II handeln. Vorzugsweise handelt es sich um Diabetes Typ II. Ferner werden die vorliegend beschriebenen Zusammensetzungen allgemein für die Behandlung und/oder Prophylaxe solcher Erkrankungen und Zustände vorgeschlagen, bei denen es vorteilhaft ist, die Zuckeraufnahme aus dem Darm zu verlangsamen und/oder zu reduzieren.

Die vorliegend zur Behandlung oder Prophylaxe vorgeschlagenen Zusammensetzungen können auch in vorteilhafter Weise mit anderen Wirkstoffen verwendet werden, z.B. zusammen mit anderen im Stand der Technik beschriebenen antidiabetischen Mitteln, wie z.B. Biguaniden, Sulfonylharnstoffen, Glycosidaseinhibitoren, vorzugsweise Inhibitoren von kohlenhydratspaltenden Enzymen des Verdauungstrakts (z.B. O-4,6-Dideoxy-4-[[1S-(1S, 4R, 5S, 6S)-4,5,6-trihydroxy-3-(hydroxymethyl)-2-cyclohexen-1-yl]-amino]-alpha-D-glucopyranosyl-(1->4)-O-alpha-D-glucopyranosyl-(1->4)-D-glucopyranose (siehe DE 23 47 782), Thiazolidindionen, Dipeptidylpeptidase IV (DP4)-Inhibitoren, Meglitiniden, Glucagon-ähnliches Peptid-1 (GLP-1), PTP1B-Inhibitoren, Glycogenphosphorylase-Inhibitoren, Glucose-6-Phosphatase-Inhibitoren sowie SGLT1- und SGLT2-Inhibitoren, die in den nachfolgenden Schutzrechten beschrieben werden: WO 02/080936, WO 02/080935, JP 2000080041, EP 0 850 948 (Propiophenonglckoside); WO 02/044192, WO 02/028872, WO 03/011880 und WO 01/068660 (2-Glucopyranoslyoxybenzylbenzole); WO 02/068440, WO 02/068439, WO 02/36602, WO 01/016147, WO 02/053573, WO 03/020737, WO 03/090783, WO 04/014932, WO 04/019958 und WO 04/018491 (Glucopyranosyloxypyrazole); WO 01/074835 und WO 01/074834 (O-Glycosidbenzamide) ; WO 04/007517 (Glucopyranosyloxythiophene); WO 03/099836, WO 01/027128 und US 2002/0137903 (C-Arylglycoside); DE 102 58 008 (Fluor-Glycosid-Derivate); DE 10 2004 028 241.2 (Fluor-Glycosid-Derivate von Pyrazolen).

Die erfindungsgemäß verwendeten Zusammensetzungen, die mindestens Phlorizin sowie einen oder mehrere LPH-Inhibitoren enthalten, werden vorliegend für die gemeinsame oder für die nacheinander erfolgende Verabreichung formuliert sein. Daher betrifft die vorliegende Erfindung auch die Verwendung von Phlorizin sowie einem oder mehreren LPH-Inhibitoren zur Behandlung und/oder zur Prophylaxe einer hyperglykämischen Erkrankung, wobei die Komponenten für die getrennte Verabreichung formuliert sind.

In einem Aspekt betrifft die vorliegende Erfindung die Verwendung einer wie oben beschriebenen Zusammensetzung zur Herstellung einer pharmazeutischen Zusammensetzung, eines diätetischen Lebensmittels oder eines Nahrungsergänzungsmittels.

In einem noch weiteren Aspekt betrifft die vorliegende Erfindung somit auch eine pharmazeutische Zusammensetzung, ein diätetisches Lebensmittel oder ein Nahrungsergänzungsmittel, die/das eine wie oben beschriebene Zusammensetzung umfasst, welche mindestens einen LPH-Inhibitor und Phlorizin umfasst. Die pharmazeutische Zusammensetzung, das diätetische Lebensmittel oder das Nahrungsergänzungsmittel eignen sich zur Verwendung in einem Verfahren zur Behandlung und/oder Prophylaxe einer hyperglykämischen Erkrankung, vorzugsweise ausgewählt aus der Gruppe bestehend aus Fettsucht, Diabetes oder eine von Diabetes verursachte Folgeerkrankung, und insbesondere zur Behandlung und/oder Prophylaxe der Diabetes vom Typ II.

Eine die vorliegend beschriebene Zusammensetzung umfassende pharmazeutische Zusammensetzung kann für die orale, parenterale oder topische Darreichung formuliert sein. Eine solche pharmazeutische Zusammensetzung kann mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Die pharmazeutischen Zusammensetzungen können beispielsweise in Form von Granulaten, Pulvern, Tabletten, Kapseln, Sirup, Suppositorien, Injektionen, Emulsionen, Suspensionen oder Lösungen vorliegen. Die Zusammensetzungen können für verschiedene Verabreichungsarten formuliert sein, beispielsweise für die orale, parenterale, topische, bukkale, sublinguale, transmukosale, rektale, subkutane intrathekale, intravenöse, intramuskuläre, intraperitoneale, nasale, intraokulare oder intraventrikuläre Verabreichung. Die Formulierung als orale, parenterale oder topische Zusammensetzung ist besonders bevorzugt. In einer besonders bevorzugten Ausführungsform ist die Zusammensetzung für die orale Verabreichung formuliert. Die pharmazeutischen Zusammensetzungen können auch als Retardmittel formuliert werden.

Für die orale, bukkale und sublinguale Verabreichung werden in der Regel feste Formulierungen wie, z.B. Pulver, Suspensionen, Granulate, Tabletten, Pillen, Kapseln und Gelcaps verwendet.

Diese können beispielsweise hergestellt werden, indem man die wirksamen Bestandteile (das Phlorizin und den einen oder die mehreren Inhibitoren der Laktase-Phlorizin-Hydrolase) mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropylmethylcellulose, Casein, Albumin, Mannit, Dextran, Saccharose, Lactose, Sorbitol, Stärke, Agar, Alginate, Pectine, Kollagen, Glyceride oder Gelatine verwendet werden. Ferner können Zusammensetzungen für die orale Verabreichung Antioxidantien (z.B. Ascorbinsäure, Tocopherol oder Cystein), Gleitmittel (z.B. Magnesiumstearat), Konservierungsmittel (z.B. Paraben oder Sorbinsäure), Geschmacksverbesserer, Sprengmittel, Bindemittel, Verdicker, Farbstoffe und ähnliche Substanzen umfassen.

Flüssige Formulierungen der vorliegend beschriebenen Zusammensetzungen, die sich für die orale Verabreichung eignen, können beispielsweise als Emulsion, Sirup, Suspension oder Lösungen vorliegen. Diese Formulierungen können unter Verwendung einer sterilen Flüssigkeit als Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Für den Gebrauch in flüssigen Dosisformen geeignete Öle umfassen beispielsweise Olivenöl, Sesamöl, Erdnussöl, Rapsöl und Maisöl. Geeignete Alkohole umfassen Ethanol, Isopropylalkohol, Hexadecylalkohol, Glycerin und Propylenglycol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

Die vorliegende Erfindung betrifft ferner auch ein diätetisches Lebensmittel oder ein Nahrungsergänzungsmittel, das eine wie oben definierte Zusammensetzung umfasst, d.h. eine Zusammensetzung, die Phlorizin und einen oder mehrere Inhibitoren der LPH enthält. Das diätetisches Lebensmittel oder Nahrungsergänzungsmittel ist insbesondere für die Behandlung und/oder Prophylaxe der wie oben definierten Fettsucht, Diabetes (insbesondere Diabetes Typ II) oder einer von Diabetes verursachten Folgeerkrankung geeignet. Das Phlorizin kann aus mindestens einem Phlorizin-haltigen, pflanzlichen Extrakt stammen. Der Extrakt stammt vorzugsweise von einer Pflanze der Gattung *Malus,* insbesondere aus einer Pflanze der Spezies *Malus domesticus.* Es ist besonders bevorzugt, dass mindestens ein Extrakt aus einer Pflanze der Gattung *Malus* aus den Äpfeln oder der Rinde einer Malus-Pflanze gewonnen wurde.

### BEISPIELE

Die nachfolgenden Beispiele beschreiben die Wirkung der vorliegend beschriebenen Zusammensetzungen:

**Tabelle 1: Inhibition verschiedener Glycosidasen durch Quercetin- und Kaempferolderivate; nb: nicht bestimmt.**

| **Nr.** | **Name Inhibitor** | **Synonyme** | **Inhibition der LPH (10 µg/ml Inhibitor)** | **Inhibition der LPH (50 µg/ml Inhibitor)** |
|---|---|---|---|---|
| 1 | Quercitrin | Quercetin-3-0-rhamnosid | nb | 12,8% (100µg/ml) |
| 2 | Quercetin-3-glucopyrano sid | Quercetin-3-O-glucopyranosid | nb | 13,7% (100µg/ml) |
| 3 | Quercetin-7-glucosid | 3,3',4',5,7-Pentahydroxyflav on-7-glucosid | **44,0%** | **69,4%** |
| 4 | Kaempferol-3-0-glucosid | Astragalin | 3,3% | 45% |
| 5 | Kaempferol-7-0-glucosid | Populnin | **53,0%** | **70,5%** |
| 6 | Kaempferol-3-0-rutinosid | Nicotiflorin | (+7,2%) | 6,6% |
| 7 | HYPEROSID | Quercetin-3-galactosid | 18,4% | 26,2% |
| 8 | SPIRAEOSID | Quercetin-4'-glucosid | 31,3% | 44,1% |
| 9 | RUTIN | Quercetin-3-O-rutinosid Quercetin-3-O-glucorhamnosid | 34,7% | 36,0% |
| 10 | KAEMPFERITR IN | Kaempferol-3,7-di-O-α-L-rhamnosid; 3,4',5,7-Tetrahydroxyflav on-3,7-dirhamnosid | 19,8% | 18,4% |
| 11 | QUERCETIN-3-GLUCOSID 5,00 | 3-Glucosylquerceti n; 3,3',4',5,7-Pentahydroxyflav on-3-glucosid | 21,6% | 38,6% |
| 12 | KAEMPFEROL-3-GLUCOSID | 3-Glucosylkaempfer ol; 3,4',5,7-Tetrahydroxyflav on-3-glucosid | 18,5% | 11,7% |

### Beispiel 2: Inhibitorische Wirkung einer Kombination aus Phlorizin und LPH-Inhibitor auf LPH/SGLT1

Die Wirkung einer Kombination von Phlorizin und eines LPH-Inhibitors auf die Aufnahme von Glucose durch den SGLT 1-Transporter wurde mit einem *ex vivo* Modell getestet, das die Situation im menschlichen Darm simuliert. Das *ex vivo* Testmodell verwendet humanes Dünndarmgewebe, das die Laktase-Phlorizin-Hydrolase (LPH) exprimiert und somit in der Lage ist, Phlorizin und andere Glycoside zu spalten. Anschließend wurden die in dieser Weise vorprozessierten Versuchsansätze in einem *in vitro* Assay auf ihre inhibitorische Wirkung auf den SGLT1 Transporter getestet.

Humanes Dünndarmgewebe, das die Laktase-Phlorizin-Hydrolase (LPH) exprimiert, wurde wie in Amiri & Naim beschrieben, hergestellt und verwendet (J Inherit Metab Dis (2012) 35:949-954). Zusammenfassend wurden sogenannte "brush border membranes" aus dem Dünndarm hergestellt und in einem entsprechenden Puffer mit Phlorizin und/oder dem jeweiligen LPH-Inhibitor 90 Minuten inkubiert.

Anschließend wurden die Dünndarmmembranen abgetrennt und die Aktivität des SGLT1 in den Überständen nach einem durch Kottra & Daniel beschriebenen, elektrophysiologischen Verfahren bestimmt (J Pharmacol Exp Ther. 2007; 322 (2) :829-35) . Hierbei wurden Xenopus-Oocyten eingesetzt, die den humanen SGLT1 exprimieren. Der Transport der Glucose durch SGLT1 kann gemessen werden, da der gemeinsame Transport von Glucose und Natrium durch SGLT1 einen Strom durch die Oocytenmembran bewirkt. Dieser wird elektrophysiologisch mittels eines "Zwei-Elektroden-Voltage-Clamp"-Verfahrens bestimmt. Eine Reduktion der Aktivität des SGLT1 führt in diesem Modell auch zu einer Verringerung des Stroms über die Zellmembran.

### Ergebnisse:

Es konnte gezeigt werden, dass Phlorizin und die getesteten LPH-Inhibitoren die Aktivität des SGLT in synergistischer Weise reduzieren. Tabelle 2 zeigt die Inhibition durch die getesteten Verbindungen mit den jeweiligen Inhibitionswerten in Prozent der Ursprungsaktivität. Überraschenderweise war die Kombination von Phlorizin und den getesteten LPH-Inhibitoren Kaempferol-7-O-Glucosid bzw. Quercetin-7-Glucosid, jeweils in der Lage, die Aktivität des SGLT deutlich stärker zu reduzieren als die einzelnen Substanzen in Summe (siehe z.B. Ansatz A). Während Phlorizin, Quercetin-7-Glucosid und Kaempferol-7-O-Glucosid alleine jeweils die Glucoseaufnahme um 32%, 2% bzw. 2,5% reduzierten, waren die Kombinationen aus Phlorizin und Quercetin-7-Glucosid bzw. aus Phlorizin und Kaempferol-7-0-Glucosid in der Lage, die Glucoseaufnahme um 48% bzw. 52% zu reduzieren.

Derselbe Effekt war ebenfalls zu beobachten, wenn statt des reinen Phlorizins ein Apfelextrakt (Ansatz B) eingesetzt wurde, der 15% Phlorizin enthält. Dieser Extrakt inhibierte die Aktivität des SGLT um 33%. Quercetin-7-Glucosid und Kaempferol-7-Glucosid allein reduzierten in diesem Ansatz die Aktivität von SGLT1 um 2,5% bzw. 2,0%. Die Kombination des Apfelextraktes mit Quercetin-7-Glucosid oder Kaempferol-7-Glucosid erzielte dagegen eine Inhibition um 49% bzw. 54%.

**Tabelle 2: Inhibition der durch SGLT1-vermittelten Glucoseaufnahme in einem ex vivo Modell**

| **Ansatz A** | **Glucoseaufnahme, Inhibition** |
|---|---|
| Phlorizin 0,3 µg/ml | 32% |
| Quercetin-7-Glucosid 10 µg/ml | 2% |
| Quercetin-7-Glucosid 10 µg/ml **&** Phlorizin 0,3 µg/ml | 48% |
| Kaempferol-7-O-Glucosid 10 µg/ml | 2,5% |
| Kaempferol-7-O-Glucosid 10 µg/ml **&** Phlorizin 0,3 µg/ml | 52% |
| **Ansatz B** | |
| Apfelextrakt (mit 15% Phlorizin) 2 mg/ml | 33% |
| Quercetin-7-Glucosid 10µg/ml | 2,5% |
| Apfelextrakt (mit 15% Phlorizin) 2 mg/ml **&** Quercetin-7-Glucosid 10 µg/ml | 49% |
| Kaempferol-7-O-Glucosid 10 µg/ml | 2,0% |
| Apfelextrakt (mit 15% Phlorizin) 2 mg/ml **&** Kaempferol-7-O-Glucosid 10 µg/ml | 54% |

## Patentansprüche

1. Zusammensetzung zur Verwendung in einem Verfahren zur Behandlung und/oder Prophylaxe einer hyperglykämischen Erkrankung, wobei die Zusammensetzung folgendes umfasst:
(a) Phlorizin; und
(b) einen oder mehrere Inhibitoren der Laktase-Phlorizin-Hydrolase, wobei der Inhibitor ein Glycosid eines Flavonols mit der Struktur gemäß Formel I 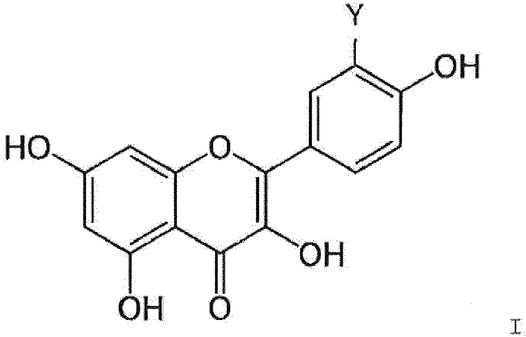 ist, in der Y ausgewählt ist aus der Gruppe bestehend aus H und OH; und
wobei der Inhibitor mindestens eine glycosidisch an das Flavonol gebundene Zucker-Gruppe aufweist, und
wobei die Zusammensetzung von 0,1 bis 10 g des Inhibitors der Laktase-Phlorizin-Hydrolase und von 0,01 bis 10 g Phlorizin umfasst.

2. Zusammensetzung zur Verwendung in einem Verfahren nach Anspruch 1, wobei sich die glycosidisch gebundene Gruppe des Inhibitors in Position 3 oder Position 7 des Flavonols befindet, wobei die glycosidisch gebundene Gruppe vorzugsweise beta-glycosidisch gebunden ist.

3. Zusammensetzung zur Verwendung in einem Verfahren nach Anspruch 1-2, wobei die Zucker-Gruppe eine Glucose-Gruppe ist.

4. Zusammensetzung zur Verwendung in einem Verfahren nach Anspruch 2-3, wobei zusätzlich zu der glycosidisch gebundenen Gruppe in Position 3 oder Position 7 des Flavonols keine weitere glycosidisch gebundene Gruppe vorhanden ist.

5. Zusammensetzung zur Verwendung in einem Verfahren nach Anspruch 4, wobei der Inhibitor der Laktase-Phlorizin-Hydrolase ausgewählt ist aus der Gruppe bestehend aus Quercetin-7-O-Glucosid und Kaempferol-7-O-Glucosid.

6. Zusammensetzung zur Verwendung in einem Verfahren nach einem der Ansprüche 1-5, wobei die Zusammensetzung von 0,01 bis 1 g Phlorizin umfasst.

7. Zusammensetzung zur Verwendung in einem Verfahren nach einem der Ansprüche 1-6, wobei die Zusammensetzung von 1 bis 10 g des Inhibitors der Laktase-Phlorizin-Hydrolase umfasst.

8. Zusammensetzung zur Verwendung in einem Verfahren gemäß einem der Ansprüche 1-7, wobei die Erkrankung vorzugsweise ausgewählt ist aus der Gruppe bestehend aus Fettsucht, Diabetes oder von Diabetes verursachten Folgeerkrankungen.

9. Verwendung einer Zusammensetzung, umfassend:
(a) Phlorizin; und
(b) einen oder mehrere Inhibitoren der Laktase-Phlorizin-Hydrolase, wobei der Inhibitor ein Glycosid eines Flavonols mit der Struktur gemäß Formel I 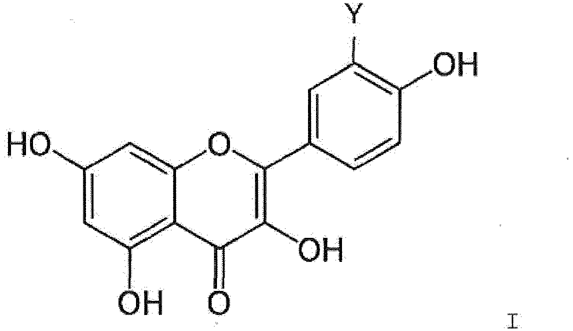 ist, in der Y ausgewählt ist aus der Gruppe bestehend aus H und OH; und
wobei der Inhibitor mindestens eine glycosidisch an das Flavonol gebundene Zucker-Gruppe aufweist, und
wobei die Zusammensetzung von 0,1 bis 10 g des Inhibitors der Laktase-Phlorizin-Hydrolase und von 0,01 bis 10 g Phlorizin umfasst,
zur Herstellung einer pharmazeutischen Zusammensetzung oder eines Nahrungsergänzungsmittels.

10. Pharmazeutische Zusammensetzung, diätetisches Lebensmittel oder Nahrungsergänzungsmittel, die/das eine Zusammensetzung nach einem der Ansprüche 1-7 umfasst, zur Verwendung in einem Verfahren zur Behandlung und/oder Prophylaxe einer hyperglykämischen Erkrankung.

11. Pharmazeutische Zusammensetzung, diätetisches Lebensmittel oder Nahrungsergänzungsmittel zur Verwendung in einem Verfahren nach Anspruch 10, wobei die hyperglykämische Erkrankung ausgewählt ist aus der Gruppe bestehend aus Fettsucht, Diabetes oder eine von Diabetes verursachte Folgeerkrankung.

12. Zusammensetzung zur Verwendung nach einem der Ansprüche 1-8, oder pharmazeutische Zusammensetzung, diätetisches Lebensmittel oder Nahrungsergänzungsmittel zur Verwendung nach einem der Ansprüche 10-11, wobei der Diabetes vom Typ II ist.

13. Pharmazeutische Zusammensetzung zur Verwendung nach Anspruch 11 oder 12, **dadurch gekennzeichnet, dass** sie für die orale, parenterale oder topische Darreichung formuliert ist.

## Claims

1. Composition for use in a method of treatment and/or prophylaxis of a hyperglycemic disease, wherein said composition comprising
(a) phlorizin; and
(b) one or more inhibitors of lactase-phlorizin-hydrolase, wherein the inhibitor is a glycoside of a flavonol having the structure of formula I 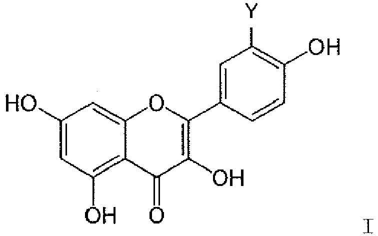 wherein Y is selected from the group consisting of H and OH; and
wherein the inhibitor comprises at least one sugar group which is glycosidically linked to the flavonol, and
wherein the composition comprises from 0.1 to 10 g of the inhibitor of lactase-phlorizin-hydrolase and from 0.01 to 10 g of phlorizin.

2. Composition for use in a method of claim 1, wherein the glycosidically bonded group of the inhibitor is located at position 3 or position 7 of the flavonol, wherein the glycosidically bonded group is preferably beta-glycosidically bonded.

3. Composition for use in a method of claims 1-2, wherein the sugar group is a glucose group.

4. Composition for use in a method of claims 2-3, wherein no glycosidically bonded group is present in addition to the glycosidically bonded group in position 3 or position 7 of the flavonol.

5. Composition for use in a method of claim 4, wherein the inhibitor of lactase phlorizin hydrolase is selected from the group consisting of quercetin-7-O-glucoside and kaempferol-7-O-glucoside.

6. Composition for use in a method of any one of claims 1-5, wherein the composition comprises from 0.01 to 1 g of phlorizin.

7. Composition for use in a method of any one of claims 1-6, wherein the composition comprises from 1 to 10 g of the inhibitor of lactase-phlorizin-hydrolase.

8. Composition for use in a method of any one of claims 1-7, wherein the disease is preferably selected from the group consisting of obesity, diabetes or secondary diseases caused by diabetes.

9. Use of a composition comprising
(a) phlorizin; and
(b) one or more inhibitors of lactase-phlorizin-hydrolase, wherein the inhibitor is a glycoside of a flavonol having the structure of to formula I 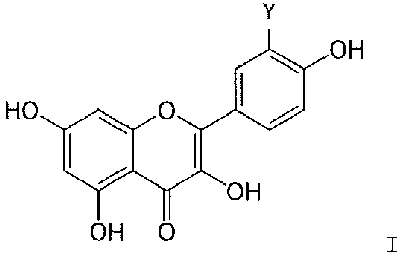 wherein Y is selected from the group consisting of H and OH; and
wherein the inhibitor comprises at least one sugar group which is glycosidically linked to the flavonol, and
wherein the composition comprises from 0.1 to 10 g of the inhibitor of lactase-phlorizin-hydrolase and from 0.01 to 10 g of phlorizin,
for the preparation of a pharmaceutical composition or a nutritional supplement.

10. Pharmaceutical composition, dietary food or nutritional supplement comprising a composition of any one of claims 1-7 for use in a method of treatment and/or prophylaxis of a hyperglycemic disease.

11. Pharmaceutical composition, dietary food or nutritional supplement for use in a method according to claim 10, wherein the hyperglycemic disease is selected from the group consisting of obesity, diabetes or a secondary disease caused by diabetes.

12. Composition for use according to any one of claims 1-8, or a pharmaceutical composition, dietary food or nutritional supplement for use according to any one of claims 10-11, wherein the diabetes is type II diabetes.

13. Pharmaceutical composition for use according to claim 11 or 12, wherein the composition is formulated for oral, parenteral or topical administration.

## Revendications

1. Composition pour utilisation dans un procédé de traitement et/ou de prophylaxie d'une maladie hyperglycémique, laquelle composition comprend ce qui suit :
a) de la phloridzine,
b) et un ou plusieurs inhibiteurs de la lactase phloridzine hydrolase, lequel inhibiteur est un glycoside d'un flavonol présentant la structure indiquée dans la formule I : 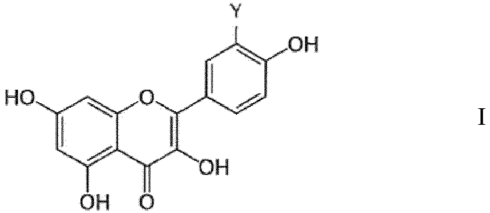 dans laquelle Y représente une entité choisie dans l'ensemble constitué par un atome d'hydrogène et un groupe hydroxyle, étant entendu que cet inhibiteur comporte au moins un groupe dérivé d'un sucre, lié au flavonol par une liaison glycosidique,
laquelle composition comprend de 0,1 à 10 g de l'inhibiteur de lactase phloridzine hydrolase et de 0,01 à 10 g de phloridzine.

2. Composition pour utilisation dans un procédé, conforme à la revendication 1, dans laquelle le groupe lié par liaison glycosidique de l'inhibiteur se trouve en position 3 ou en position 7 du flavonol, et le groupe lié par liaison glycosidique est de préférence lié par une liaison bêta-glycosidique.

3. Composition pour utilisation dans un procédé, conforme à la revendication 1 ou 2, dans laquelle le groupe dérivé d'un sucre est un groupe dérivé du glucose.

4. Composition pour utilisation dans un procédé, conforme à la revendication 2 ou 3, dans laquelle, hormis le groupe lié par liaison glycosidique en position 3 ou en position 7 du flavonol, il n'y a aucun autre groupe lié par liaison glycosidique.

5. Composition pour utilisation dans un procédé, conforme à la revendication 4, dans laquelle l'inhibiteur de lactase phloridzine hydrolase est choisi dans l'ensemble constitué par le 7-O-glucoside de quercétine et le 7-O-glucoside de kaempférol.

6. Composition pour utilisation dans un procédé, conforme à l'une des revendications 1 à 5, laquelle composition comprend de 0,01 à 1 g de phloridzine.

7. Composition pour utilisation dans un procédé, conforme à l'une des revendications 1 à 6, laquelle composition comprend de 1 à 10 g de l'inhibiteur de lactase phloridzine hydrolase.

8. Composition pour utilisation dans un procédé, conforme à l'une des revendications 1 à 7, pour laquelle la maladie est choisie de préférence dans l'ensemble constitué par l'adipose, le diabète et les maladies consécutives à un diabète.

9. Utilisation d'une composition comprenant :
a) de la phloridzine,
b) et un ou plusieurs inhibiteurs de la lactase phloridzine hydrolase, lequel inhibiteur est un glycoside d'un flavonol présentant la structure indiquée dans la formule I : 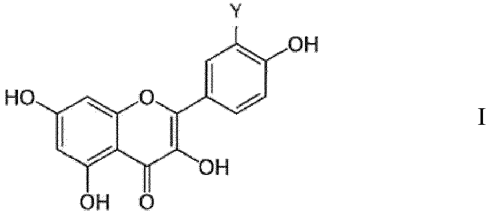 dans laquelle Y représente une entité choisie dans l'ensemble constitué par un atome d'hydrogène et un groupe hydroxyle, étant entendu que cet inhibiteur comporte au moins un groupe dérivé d'un sucre, lié au flavonol par une liaison glycosidique,
laquelle composition comprend de 0,1 à 10 g de l'inhibiteur de lactase phloridzine hydrolase et de 0,01 à 10 g de phloridzine,
pour la fabrication d'une composition pharmaceutique ou d'un complément nutritionnel.

10. Composition pharmaceutique, aliment diététique ou complément nutritionnel, comprenant une composition conforme à l'une des revendications 1 à 7, pour utilisation dans un procédé de traitement et/ou de prophylaxie d'une maladie hyperglycémique.

11. Composition pharmaceutique, aliment diététique ou complément nutritionnel pour utilisation dans un procédé, conforme à la revendication 10, pour laquelle ou lequel la maladie hyperglycémique est choisie dans l'ensemble constitué par l'adipose, le diabète et une maladie consécutive à un diabète.

12. Composition pour utilisation, conforme à l'une des revendications 1 à 8, ou composition pharmaceutique, aliment diététique ou complément nutritionnel pour utilisation dans un procédé, conforme à l'une des revendications 10 et 11, pour laquelle ou lequel le diabète est un diabète de type II.

13. Composition pharmaceutique pour utilisation, conforme à la revendication 11 ou 12, **caractérisée en ce qu'**elle est formulée pour administration par voie orale, parentérale ou topique.
